# EUROPEAN PATENT APPLICATION

(11) **EP 3 862 441 A2**
(43) Date of publication of application: **11.08.2021**
(21) Application number: 21152982.1
(22) Date of filing: 22.01.2021
(51) Int. Cl.: C12Q 1/6886

(54) **METHOD FOR THE EARLY DIAGNOSIS OF CANCER BY MEANS OF DDPCR ANALYSIS OF MIRNA AND PROTEIN MARKERS IN LIQUID BIOPSY**

(30) Priority: 04.02.2020 IT 202000002107
(71) Applicant: Artemisia S.p.A., 00198 Roma (RM) (IT)
(72) Inventor: GIORLANDINO, CLAUDIO, I-00198 ROMA (RM) (IT); LONGO, SALVATORE, I-00198 ROMA (RM) (IT); MARGIOTTI, KATIA, I-00198 ROMA (RM) (IT); MESORACA, ALVARO, I-00198 ROMA (RM) (IT); RAMOS CIRILLO, PRISCILA DANIELE, I-00198 ROMA (RM) (IT)
(74) Representative: Fiammenghi, Eva

(57) **Abstract**

Non-invasive diagnosis method for the early determination of cancer by means of liquid biopsy, said method being based on the identification, in a peripheral blood sample previously drawn from the patient, of the expression profile of circulating miRNA and of serum proteins released in the blood circulation by solid tumors, said method also considering markers such as age and sex and allowing the early analysis of a tumoral pathological state in initial phase.

## Description

### Field of the art

The present invention regards a non-invasive test for the early diagnosis of cancer by means of analysis of the expression profile of circulating miRNA combined with the analysis of bioprotein markers in the patient. The combined analysis is based on the use of a mathematical model of different assays that have resulted highly specific for liver tumor, lung tumor, ovary tumor, pancreatic tumor and stomach tumor in early phase.

### Prior art

During tumor progression, selective pressure leads to the emergence of subclonal populations of tumor cells which are replicated and diffused in a more efficient manner. During this process, DNA, microRNA (miRNA) and proteins contained in the tumor cells are released in the blood circulation. Therefore, the possibility of identifying specific mutations of cancer and/or other tumor biomarkers in the body fluids is becoming an interesting field of application of oncology: the liquid biopsy. Through the liquid biopsy it is possible to analyze/quantify the nucleic acids and the tumor proteins which can be used as markers for monitoring the response to the treatment, evaluating the appearance of resistance to drugs, quantifying the minimum residual disease and in the early diagnosis of cancer¹. The early diagnosis of cancer is one of the keys for reducing cancer mortality. Most solid tumors, if diagnosed in the initial phases of development, can be treated, thus reducing the possibility of being diffused as remote metastasis^{2,3}. Initiatives such as GRAIL and Thrive Earlier Detection are using "multi-analyte" strategies for the early diagnosis of various solid tumors, including ovarian carcinoma, lung carcinoma, pancreas carcinoma, liver carcinoma and stomach carcinoma⁴. Nevertheless, all these studies are concentrating on broad spectrum methods such as the Next Generation Sequencing (NGS), based on screening of specific mutations and/or methylation models of the circulating tumor DNA (ctDNA) combined with serum proteins or not. But which method to use? Currently, all the methods used by the abovementioned studies and in particular NGS have failed due to the low specificity of such method and due to the genetic target against which they are directed. In fact these seek somatic mutations in the circulating blood, which we know to be extremely variable and tardy in their appearance in the blood of patients. In addition, these methods have high costs and have not yet produced effective tests. The method according to the present invention is a test based on the target originated from the review of the literature and database for the circulation of miRNA and proteins identified in patient with ovarian cancer, lung cancer, pancreas cancer, liver cancer and stomach cancer also in the initial phases. A personalized ddPCR dosage panel is designed for detecting these miRNA and proteins. The personalized exams have their pros and cons: Pros: 1) with few objectives, the cost tends to be low for the final client; 2) ddPCR is the most sensitive updated platform for the low quantity of circulating nucleic acids. Cons: 1) A test based on target should have high specificity and sensitivity; 2) This is not a "classic" wide spectrum screening test, hence other important miRNAs and/or proteins might not be considered; 3) Cancer is a heterogeneous disease and the miRNAs and the proteins are not the only things involved in its development (point-like mutations, variation of the number of copies, differential gene expression, epigenetic regulation, etc.)¹⁸. The studies and experiments performed by the research team of the Applicant, in order to overcome the preceding problems have developed a strategy based on PCR. Such technique is termed "digital PCR". The droplet digital PCR (ddPCR) is presently the standard method for monitoring the response to therapy in the patients with lung carcinoma and colon-rectal carcinoma^{6,7}. Up to now ddPCR is the most sensitive technology for detecting rare mutations, but also for the early diagnosis of cancer. The applications of ddPCR for the early study of cancer are particularly complicated to design, especially because the identification of a panel of specific biomarkers, which result altered both in the early phase and in the tardy phase of the tumor, is complex. Up to now, it is known that the diagnosis of the tumor requires a series of laboratory and image exams, including the biopsy of the tissues, which is the "gold standard" technique for the classification of the tumor. These methods are highly invasive and costly. In recent years, the scientific community relied on the conventional diagnostic protocols for the diagnosis, the prognosis and the therapeutic evaluation of cancer patients. The routine tests on the tumor tissue samples have proven useful for various applications, however there are several limitations, such as the incapacity to accurately monitor the minimum residual disease and the invasiveness of the solid biopsy, which can also be extremely painful for patients. Up to now, the routine measurement of the CA-125, carcinoembryonic antigen (CEA) and prostate-specific antigen (PSA) are the only circulating biomarkers used for the early diagnosis of several types of cancer, but their use and diagnostic power is still the object of lengthy discussions¹⁵. The progresses in the area of molecular diagnostics and the possibility of subjecting tissues or blood samples to screening in order to define genomic profiles, proteomic profiles and epigenetic profiles specific for the tumor have stimulated the search for biomarkers capable of detecting the presence of tumors even in the first development phases. Liquid biopsy is emerging as possible "futuristic test" in the field of oncology, but presently it is still hard to use the liquid biopsy tests for the purpose of early diagnosis. Their application is mainly adapted for monitoring the treatment, where the patient could have a metastatic disease with high content of tumor proteins, RNA and DNA released in the blood circulation. In addition the liquid biopsy does not substitute the tissue biopsy, which is always necessary for the diagnosis, but complementary for identifying prognostic or predictive markers.

Up to now, ddPCR - even if considered the most important technology for detecting tumor biomarkers - has not produced valid applications for early diagnosis. The miRNA are fragments of non-coding single-stranded RNA (ncRNA) with 19-25 nucleotides and it is assumed that they regulate more than 50% of all the genes coding the proteins, given that they are regulators of the overall gene expression of the genome⁹. The miRNA are released in the blood circulation mainly by the secretion of active cells (e.g. proliferating tumor cells) through microvesicles and/or exosomes and have already been described as involved in the formation of pre-metastatic niches⁹. In addition, many studies have demonstrated that ddPCR is an excellent approach for quantifying low quantities of circulating miRNA in the fluids in the blood like serum¹⁰¹¹¹².

### Description of the invention

The object of the present description is a new non-invasive method for the early diagnosis of cancer by means of liquid biopsy. More in detail the present description regards an innovative diagnostic test based on liquid biopsy for the detection of the expression profile of circulating miRNA combined with the search for the serum proteins released in the blood circulation by solid tumors. By concentrating on the combined analysis of the expression of miRNA and of proteins, the objective of this test is the detection of cancer also in very early phases.

Still more specifically, the present invention regards a non-invasive test for the early diagnosis of cancer in which the sample of the patient is subjected to a profile of circulating miRNA combined with the analysis of the proteins. The combined analysis is based on the use of a mathematical model of the different assays that resulted highly specific for the liver tumor, lung tumor, ovary tumor, pancreatic tumor and stomach tumor in early phase. The achievement of the present invention arises from in-depth preliminary studies that start from the recognition that the proteins, which are produced both by the normal cells and by the tumor cells but in much higher quantities by the tumor cells, are released in the blood flow by the active tumor cells¹³.

The idea underlying the present invention is therefore the joint identification of profiles of miRNA and proteins in the blood circulation which turns out to be an effective information test for the evidence of cancer even in the initial phases.

### Limits of the assay:

a) **Pre-analytical:** With regard to the sources of miRNA, plasma and serum are indicated, but in the plasma the specific signal of the disease could be masked by the miRNA contained in the platelets. In addition to the platelet contamination, the quantification of the miRNA could be affected by the presence of erythrocyte contamination and hemolysis, since much miRNA are present in the erythrocytes¹⁶. Hence, it is preferred to execute the test on serum. For the proteins, it is necessary a single blood drawing in order to obtain, after centrifugation, serum samples used for quantifying the biomarkers. The test tubes used in the experiments relative to the present invention do not require any type of anti-coagulant but the centrifugation must occur within 2 hours from the blood drawing.
b) **Normalization of the data of the expression of the circulating miRNA:** since the miRNA are often present at very low concentrations in the serum and in the plasma, their quantification requires, for example, highly sensitive and specific ddPCR methods. In addition, several technical variables introduced during the experimental phases (quantity of initial sample, collection mode, conditions of preservation and isolation / efficiency of transcription of the miRNA) significantly influence the final quantity of miRNA, in the end masking the actual biological response. The object of the normalization is to reduce the analytical variability so as to obtain the most reliable and reproducible biological result. Therefore, the selection of the appropriate normalizer(s) is a crucial aspect in the quantification of the miRNA, above all for their implementation in a clinical setting. Among the normalization strategies used for the profile of the circulating miRNA in the scope of the present invention, the addition of an exogenous synthetic miRNA has been selected for the inter and intra-test evaluation. Nevertheless, the endogenous miRNA are considered the optimal reference targets, since their expression is influenced by the same physiological and pathogenetic conditions. Up to now, there exists no universally accepted method on the best normalization strategy for the circulating serum miRNA. This was validated during the development of our assay.
c) **Validation in longitudinal studies:** a test for early detection of cancer must be carefully designed and validated in order to prevent results of false positives that generate worry and additional follow-up examinations that are not necessary (spending time and money of patients and health operators) and preventing false negative results. A suitable technical and clinical validation was therefore executed so that the liquid biopsy can be implemented as instrument for the early diagnosis of cancer.

### Detailed description of the invention

### Analytical description

The method according to the present invention comprises the following passages:
- Purification of miRNA and proteins from blood;
- Reverse transcription of the total miRNA;
- Quantification of miRNA by means of ddPCR;
- Quantification of the serum proteins;
- Statistical analysis for the determination of the diagnostic indices.

### More in detail:

### Passage 1: Purification of miRNA from blood.

Donors not affected by tumor were selected based on cancer family history, sex and age. Blood drawing was executed by using the Vacutest (clot activator) test tubes and/or BD Vacutainer SST (gel bed) without anti-coagulants (like EDTA and citrate). The test tubes were centrifuged for 10 minutes at 1900 x g (3000 rpm) and 4 °C. The serum was transferred into a new 2 ml test tube. For the analysis of the proteins, 1 mL was preserved in this phase (-80 °C). For the analysis of the miRNA, the serum (1 ml) was once again centrifuged for 10 minutes at 16000 x g and 4 °C. The serum was accurately transferred into a new 2 ml test tube and maintained at -80 °C up to the collection of miRNA. Serum samples of patients with solid tumors in phases I-IV were acquired at the Victorian Cancer Biobank (Australia, Melbourne, VIC) with ethical approval. Upon arrival, the serum (500uL) was preserved at -80 °C. The purification of miRNA was executed in accordance with the instructions of the protocol of the producer, with several modifications (miRNeasy Serum / Plasma Advanced Kit-QIAGEN).

### Passage 2: Universal PCR reaction with reverse transcription (RT) of miRNA.

Four microliters of miRNA were retrotranscribed by using the kit for synthesizing cDNA, miRCURY LNA RT Kit (QIAGEN). Then, 9uL of cDNA (previously diluted 1:50 or 1:500) were prepared for the amplification in a reaction volume of 20uL containing 10 uL 2X DNA binding-dye EVA Green Supermix (BioRad) and 1 uL miRCURY LNA miRNA PCR Assay (QIAGEN).

### Passage 3: droplet digital PCR work flow

Each mixture for the assay ddPCR (20uL) was loaded in a cartridge of the disposable droplet generator (BioRad). Then, 70 uL of droplet generation oil (BioRad) were loaded in each of the eight oil wells. The cartridge was then positioned within the droplet generator QX200 (BioRad). At the end of the droplet generation, the droplets were transferred onto a PCR plate with 96 wells (BioRad) by using a multichannel pipette. The plate was heat-sealed with a sheet and placed in a normal thermal cycler. The PCR conditions were the following: 95 °C for 5 minutes, hence 40 cycles at 95 °C for 30 seconds and 58 °C for 1 minute (ramp speed reduced to 1.6%) and a phase for stabilizing the final signal at 4 °C for 5 minutes and 90 °C for 5 minutes. A no template control (NTC) and a negative control for each reverse transcription (RT-neg) reaction were included in each dose. The thresholds ddPCR for each dose were defined by using acceptation criteria, which were defined during optimization. The software QuantaSoft ™ Analysis Pro (version 1.0.596) or QuantaSoft ™ (version 1.7.4) were used for assigning positive / negative droplets and converting the counts into copies / uL. The quantification of each miRNA target was normalized by using the concentration of endogenous and/or exogenous miRNA.

### Passage 4: Work flow for the quantification of the proteins

For the proteins, we based ourselves on the review of the meta-analysis literature in order to select the proteins previously associated with the early diagnosis of liver tumors, lung tumors, ovary tumors, pancreas tumors and stomach tumors (Table 2).

For the quantification of the proteins, we used a commercialized kit validated for clinical samples, such as the chemiluminescent microparticle immunoassay, CMIA (Abbott) and nephelometry systems (Siemens Healthcare Diagnostics). In some cases, we have developed an "internal" method based on liquid chromatography. All the proteins were preserved at -80 °C.

### Passage 5: Statistical analysis

723 miRNA¹⁹ (Table 1) were evaluated for each tumor type and control type. Then, based on the obtained results, on the review of the scientific literature and gene expression database, it was possible to select circulating miRNAs and proteins associated with the early diagnosis of liver tumors, lung tumors, ovary tumors, pancreas tumors and stomach tumors (Table 2). Table 3 reports the characteristics of the assay developed by the research team of the Applicant. More specifically, the analytical models comprising specific miRNA and proteins and/or combinations thereof were identified, for the detection of specific tumors in an initial phase of the pathology development.

Before the statistical comparisons, the samples were divided into a "detection" set and a "validation" set. The "detection" set was used for selecting miRNA markers and proteins and constructing models which distinguish the pathological state from the normal state. Based on the combined optimization of miRNA and proteins, the diagnostic indices were generated by using Fisher's linear discriminant analysis. By using the set of cross validation left pending in the detection set, in the end the best diagnostic index was selected. An index score ≥ 0 indicated cancer and an index score <0 indicated the absence of cancer. The diagnostic sensitivity, the specificity, the accuracy and the area below the curve of the operating characteristics of the receiver (AOC) were calculated for each analyte on its own and for each diagnostic index in the validation set (Table 3). The statistical test and the graph were created by respectively using BRB-ArrayTools (R package) and GraphPad Prism version 8.3.0 (GraphPad). An in-house software was developed in order to generate reports through a user-friendly platform.

**Table 1. miRNAs associated with cancer**

| | | | | | | | |
|---|---|---|---|---|---|---|---|
| hsa-miR-1272 | hsa-miR-658 | hsa-miR-486 | hsa-miR-518b | hsa-miR-193b | hsa-miR-891a | hsa-miR-409-5p | hsa-miR-556-3p |
| hsa-miR-146a# | hsa-miR-648 | hsa-miR-26a | hsa-miR-503 | hsa-miR-199b | hsa-miR-1260 | hsa-miR-424 | hsa-miR-548a |
| hsa-miR-1274B | hsa-miR-668 | hsa-miR-220c | hsa-miR-509-3-5p | hsa-miR-195# | hsa-miR-190b | hsa-miR-30b | hsa-miR-587 |
| hsa-miR-1285 | hsa-miR-663B | hsa-miR-10a | hsa-miR-502 | hsa-miR-34b | hsa-miR-1227 | hsa-miR-29a | hsa-miR-1286 |
| hsa-miR-1293 | hsa-miR-720 | hsa-miR-489 | hsa-miR-516a-5p | hsa-miR-337-3p | hsa-miR-1256 | hsa-miR-485-3p | hsa-miR-596 |
| hsa-miR-1298 | hsa-miR-766 | hsa-miR-222 | hsa-miR-508 | hsa-miR-577 | hsa-miR-1250 | hsa-miR-484 | hsa-miR-621 |
| hsa-miR-200a | hsa-miR-640 | hsa-miR-105 | hsa-miR-200c | hsa-miR-33a# | hsa-miR-1205 | hsa-miR-380-3p | hsa-miR-605 |
| hsa-miR-374 | hsa-miR-875-5p | hsa-miR-221 | hsa-miR-525 | hsa-miR-645 | hsa-miR-744# | hsa-miR-299-3p | hsa-miR-548M |
| hsa-miR-371-3p | hsa-miR-133a | hsa-miR-770-5p | hsa-miR-524-5p | hsa-miR-363# | hsa-miR-638 | hsa-miR-28 | hsa-miR-541# |
| hsa-miR-325 | hsa-miR-152 | hsa-miR-29b-2# | hsa-miR-520g | hsa-miR-16-1# | hsa-miR-661 | hsa-miR-320 | hsa-miR-520c-3p |
| hsa-miR-362 | hsa-miR-551b | hsa-miR-30c-1# | hsa-miR-518e | hsa-miR-138-2# | hsa-miR-650 | hsa-miR-376c | hsa-miR-519b-3p |
| hsa-miR-516b | hsa-miR-185 | hsa-miR-432# | hsa-miR-520f | hsa-miR-148b# | hsa-miR-7-2# | hsa-miR-423-5p | hsa-miR-548K |
| hsa-miR-19b | hsa-miR-191 | hsa-miR-32# | hsa-miR-301b | hsa-miR-569 | hsa-miR-639 | hsa-miR-422a | hsa-miR-548H |
| hsa-miR-331 | hsa-miR-133b | hsa-miR-454# | hsa-miR-941 | hsa-miR-144# | hsa-miR-146a | hsa-miR-377 | hsa-miR-552 |
| hsa-miR-136 | hsa-miR-190 | hsa-miR-30d | hsa-miR-588 | hsa-miR-1264 | hsa-miR-148b | hsa-miR-378 | hsa-miR-582-3p |
| hsa-miR-362-3p | hsa-miR-885-3p | hsa-miR-1180 | hsa-miR-513C | hsa-miR-1283 | hsa-miR-127 | hsa-miR-374a# | hsa-miR-23a |
| hsa-miR-339-5p | hsa-miR-873 | hsa-1et-7b# | hsa-miR-603 | hsa-miR-220 | hsa-miR-181a | hsa-miR-424# | hsa-miR-9 |
| hsa-miR-330-5p | hsa-miR-122 | hsa-miR-26a-1# | hsa-miR-937 | hsa-miR-224 | hsa-miR-139-5p | hsa-miR-409-3p | hsa-miR-562 |
| hsa-miR-519d | hsa-miR-876-5p | hsa-miR-223# | hsa-miR-617 | hsa-miR-361 | hsa-miR-142-5p | hsa-miR-29a# | hsa-miR-551a |
| hsa-miR-19a | hsa-miR-886-3p | hsa-miR-24-1# | hsa-miR-635 | hsa-miR-212 | hsa-miR-127-5p | hsa-miR-30d# | hsa-miR-572 |
| hsa-miR-193a-3p | hsa-miR-889 | hsa-1et-7c# | hsa-miR-614 | hsa-miR-192 | hsa-miR-130b | hsa-miR-30e-3p | hsa-miR-566 |
| hsa-miR-194 | hsa-miR-92a | hsa-miR-513-5p | hsa-miR-623 | hsa-1et-7c | hsa-miR-642 | hsa-1et-7f-2# | hsa-miR-575 |
| hsa-miR-144 | hsa-miR-122# | hsa-miR-382 | hsa-miR-518f# | hsa-miR-342-5p | hsa-miR-655 | hsa-miR-106b# | hsa-miR-96# |
| hsa-miR-149# | hsa-miR-1825 | hsa-miR-449b | hsa-miR-524 | hsa-miR-20b | hsa-miR-99a | hsa-miR-23b# | hsa-miR-99a# |
| hsa-miR-1294 | hsa-miR-641 | hsa-miR-141 | hsa-miR-626 | hsa-miR-331-5p | hsa-miR-99b | hsa-miR-221# | hsa-miR-556-3p |
| hsa-miR-148a# | hsa-miR-1255A | hsa-miR-32 | hsa-miR-548G | hsa-miR-33b | hsa-miR-651 | hsa-miR-1179 | hsa-miR-548a |
| hsa-miR-942 | hsa-miR-649 | hsa-miR-298 | hsa-miR-513B | hsa-miR-369-3p | hsa-miR-885-5p | hsa-miR-27b# | hsa-miR-587 |
| hsa-miR-1302 | hsa-miR-1224-3P | hsa-miR-486-3p | hsa-miR-548N | hsa-miR-369-5p | hsa-miR-636 | hsa-miR-10a# | hsa-miR-1286 |
| hsa-miR-1291 | hsa-miR-631 | hsa-miR-29c | hsa-miR-518a-3p | hsa-miR-208 | hsa-miR-891b | hsa-miR-453 | hsa-miR-596 |
| hsa-miR-16-2# | hsa-miR-924 | hsa-miR-125a-5p | hsa-miR-629 | hsa-miR-542-5p | hsa-miR-1233 | hsa-miR-485-5p | hsa-miR-621 |
| hsa-miR-15a# | hsa-miR-92a-2# | hsa-miR-10b | hsa-miR-598 | hsa-miR-20a# | hsa-miR-1204 | hsa-miR-449 | hsa-miR-605 |
| hsa-miR-1289 | hsa-miR-935 | hsa-miR-301 | hsa-miR-597 | hsa-miR-19a# | hsa-miR-126# | hsa-miR-302b | hsa-miR-548M |
| hsa-miR-1253 | hsa-miR-944 | hsa-miR-181c | hsa-miR-615-5p | hsa-miR-135b# | hsa-miR-1247 | hsa-miR-411 | hsa-miR-541# |
| hsa-miR-1303 | hsa-miR-938 | hsa-miR-491-3p | hsa-miR-624 | hsa-miR-145# | hsa-miR-1263 | hsa-miR-324-5p | hsa-miR-520c-3p |
| hsa-miR-1203 | hsa-miR-892b | hsa-miR-219-2-3p | hsa-miR-628-5p | hsa-miR-1290 | hsa-miR-1249 | hsa-miR-30c | hsa-miR-519b-3p |
| hsa-miR-1282 | hsa-miR-767-5p | hsa-miR-215 | hsa-miR-520e | hsa-miR-361-3p | hsa-miR-1206 | hsa-miR-299-5p | hsa-miR-548K |
| hsa-miR-1257 | hsa-1et-7a | hsa-miR-27b | hsa-miR-450b-5p | hsa-miR-1296 | hsa-miR-125b-2# | hsa-miR-214 | hsa-miR-548H |
| hsa-miR-186# | hsa-miR-211 | hsa-miR-380-5p | hsa-miR-570 | hsa-miR-1305 | hsa-miR-647 | hsa-miR-492 | hsa-miR-552 |
| hsa-miR-493 | hsa-miR-579 | hsa-miR-25# | hsa-miR-544 | hsa-miR-1276 | hsa-miR-335# | hsa-miR-483-5p | hsa-miR-582-3p |
| hsa-miR-501-3p | hsa-miR-556-5p | hsa-miR-320B | hsa-miR-548a-5p | hsa-miR-1826 | hsa-miR-662 | hsa-1et-7f | hsa-miR-23a |
| hsa-miR-512-5p | hsa-miR-758 | hsa-miR-106a# | hsa-miR-548d | hsa-miR-1304 | hsa-miR-659 | hsa-1et-7e | hsa-miR-9 |
| hsa-miR-507 | hsa-miR-890 | hsa-miR-29b-1# | hsa-miR-26b | hsa-miR-1259 | hsa-miR-675 | hsa-miR-490 | hsa-miR-562 |
| hsa-miR-505 | hsa-miR-205 | hsa-miR-214# | hsa-miR-630 | hsa-miR-132# | hsa-miR-934 | hsa-miR-30a-5p | hsa-miR-551a |
| hsa-miR-518c | hsa-miR-1208 | hsa-miR-1184 | hsa-miR-586 | hsa-miR-1267 | hsa-miR-765 | hsa-miR-376a# | hsa-miR-572 |
| hsa-miR-511 | hsa-miR-564 | hsa-1et-7f-1# | hsa-miR-590-3P | hsa-miR-346 | hsa-miR-769-5p | hsa-miR-302b# | hsa-miR-566 |
| hsa-miR-518a-5p | hsa-miR-581 | hsa-miR-1197 | hsa-miR-10b# | hsa-miR-340 | hsa-miR-182 | hsa-miR-302d | hsa-miR-575 |
| hsa-miR-520d-5p | hsa-miR-555 | hsa-miR-202# | hsa-miR-517# | hsa-miR-345 | hsa-miR-150 | hsa-miR-30c-2# | hsa-miR-96# |
| hsa-miR-155 | hsa-miR-563 | hsa-miR-218-1# | hsa-miR-592 | hsa-miR-509-5p | hsa-miR-129 | hsa-miR-31# | hsa-miR-99a# |
| hsa-miR-541 | hsa-miR-583 | hsa-miR-196a# | hsa-miR-613 | hsa-miR-198 | hsa-miR-18b | hsa-miR-431# | hsa-miR-556-3p |
| hsa-miR-523 | hsa-miR-573 | hsa-miR-1178 | hsa-miR-600 | hsa-miR-203 | hsa-miR-15b | hsa-miR-100# | hsa-miR-548a |
| hsa-miR-921 | hsa-miR-554 | hsa-miR-143# | hsa-miR-5481 | hsa-miR-200b | hsa-miR-184 | hsa-miR-213 | hsa-miR-587 |
| hsa-miR-933 | hsa-miR-296 | hsa-miR-367# | hsa-miR-548L | hsa-miR-202 | hsa-miR-18a | hsa-miR-1182 | hsa-miR-1286 |
| hsa-miR-1284 | hsa-miR-1 | hsa-miR-338-5P | hsa-miR-520D-3P | hsa-miR-508-5p | hsa-miR-875-3p | hsa-1et-7a# | hsa-miR-596 |
| hsa-miR-411# | hsa-miR-216b | hsa-miR-135a | hsa-miR-500 | hsa-miR-139-3p | hsa-miR-892a | hsa-miR-193b# | hsa-miR-621 |
| hsa-miR-920 | hsa-miR-223 | hsa-miR-146b-3p | hsa-miR-519e# | hsa-miR-517a | hsa-miR-146b | hsa-miR-22# | hsa-miR-605 |
| hsa-miR-936 | hsa-miR-329 | hsa-miR-147b | hsa-miR-550 | hsa-miR-520a | hsa-miR-874 | hsa-miR-24-2# | hsa-miR-548M |
| hsa-miR-616 | hsa-miR-24 | hsa-miR-128a | hsa-miR-561 | hsa-miR-199a | hsa-miR-876-3p | hsa-miR-381 | hsa-miR-541# |
| hsa-miR-591 | hsa-miR-101 | hsa-miR-130a | hsa-miR-324-3p | hsa-miR-526b | hsa-miR-886-5p | hsa-miR-376b | hsa-miR-520c-3p |
| hsa-miR-599 | hsa-miR-515-5p | hsa-miR-143 | hsa-miR-95 | hsa-miR-196b | hsa-miR-337-5p | hsa-miR-296-3p | hsa-miR-519b-3p |
| hsa-miR-608 | hsa-miR-20a | hsa-miR-654 | hsa-miR-487b | hsa-miR-519e | hsa-miR-1245 | hsa-miR-412 | hsa-miR-548K |
| hsa-miR-501 | hsa-miR-363 | hsa-miR-106b | hsa-miR-557 | hsa-miR-191# | hsa-miR-637 | hsa-miR-429 | hsa-miR-548H |
| hsa-miR-518d-5p | hsa-miR-148a | hsa-miR-872 | hsa-miR-548P | hsa-miR-154# | hsa-miR-1244 | hsa-miR-450a | hsa-miR-552 |
| hsa-miR-506 | hsa-miR-330 | hsa-miR-125b | hsa-miR-601 | hsa-miR-151-5P | hsa-miR-1262 | hsa-miR-455 | hsa-miR-582-3p |
| hsa-miR-512-3p | hsa-miR-367 | hsa-miR-660 | hsa-miR-553 | hsa-miR-1300 | hsa-miR-551b# | hsa-miR-302c | hsa-miR-23a |
| hsa-miR-618 | hsa-miR-200a# | hsa-miR-708 | hsa-miR-571 | hsa-miR-151-3p | hsa-miR-1238 | hsa-miR-219 | hsa-miR-9 |
| hsa-miR-517b | hsa-miR-200b# | hsa-miR-483-3p | hsa-miR-518e# | hsa-miR-1265 | hsa-miR-656 | hsa-miR-23b | hsa-miR-562 |
| hsa-miR-510 | hsa-miR-192# | hsa-miR-18b# | hsa-miR-126 | hsa-miR-181a-2# | hsa-miR-9# | hsa-miR-674 | hsa-miR-551a |
| hsa-miR-539 | hsa-miR-200c# | hsa-miR-125b-1# | hsa-miR-433 | hsa-miR-182# | hsa-miR-644 | hsa-miR-17 | hsa-miR-572 |
| hsa-miR-532-3p | hsa-miR-20b# | hsa-miR-1248 | hsa-miR-452 | hsa-miR-1275 | hsa-miR-922 | hsa-1et-7g | hsa-miR-566 |
| hsa-miR-519c | hsa-miR-664 | hsa-miR-155# | hsa-miR-425-5p | hsa-miR-183# | hsa-miR-939 | hsa-miR-216a | hsa-miR-575 |
| hsa-miR-545 | hsa-miR-99b# | hsa-miR-1252 | hsa-miR-302° | hsa-miR-1270 | hsa-miR-549 | hsa-miR-219-1-3p | hsa-miR-96# |
| hsa-miR-520b | hsa-miR-17# | hsa-miR-633 | hsa-miR-375 | hsa-miR-1236 | hsa-miR-769-3p | hsa-miR-672 | hsa-miR-99a# |
| hsa-miR-519a | hsa-miR-1288 | hsa-miR-646 | hsa-miR-323-3p | hsa-miR-181c# | hsa-miR-943 | hsa-miR-302c# | hsa-miR-556-3p |
| hsa-miR-522 | hsa-miR-1271 | hsa-miR-665 | hsa-miR-410 | hsa-miR-595 | hsa-miR-548c | hsa-miR-218-2# | hsa-miR-548a |
| hsa-miR-520a# | hsa-miR-1269 | hsa-miR-657 | hsa-miR-487° | hsa-miR-18a# | hsa-miR-582-5p | hsa-miR-432 | hsa-miR-587 |
| hsa-miR-497# | hsa-miR-129# | hsa-miR-634 | hsa-miR-28-3p | hsa-miR-1243 | hsa-miR-576-3p | hsa-miR-1201 | hsa-miR-1286 |
| hsa-miR-593 | hsa-miR-1301 | hsa-miR-643 | hsa-miR-383 | hsa-1et-7b | hsa-miR-548d-5p | hsa-miR-302d# | hsa-miR-596 |
| hsa-miR-607 | hsa-miR-130b# | hsa-miR-142-3p | hsa-miR-107 | hsa-miR-499-3p | hsa-miR-326 | hsa-1et-7e# | |
| hsa-miR-606 | hsa-miR-218 | hsa-miR-138 | hsa-miR-384 | hsa-miR-494 | hsa-miR-208b | hsa-miR-488 | |
| hsa-miR-622 | hsa-miR-25 | hsa-miR-16 | hsa-miR-450b-3p | hsa-miR-504 | hsa-miR-98 | hsa-miR-23a# | |
| hsa-miR-518c# | hsa-miR-106a | hsa-miR-548b-5p | hsa-miR-374b# | hsa-miR-515-3p | hsa-miR-559 | hsa-miR-105# | |
| hsa-miR-628-3p | hsa-miR-27a | hsa-miR-147 | hsa-miR-377# | hsa-miR-517c | hsa-miR-584 | hsa-miR-26b# | |
| hsa-miR-625# | hsa-miR-199a-3p | hsa-miR-135b | hsa-miR-452# | hsa-miR-525-3p | hsa-miR-30a-3p | hsa-miR-101# | |
| hsa-miR-520h | hsa-miR-220b | hsa-miR-183 | hsa-miR-567 | hsa-miR-518f | hsa-miR-1324 | hsa-miR-19b-1# | |
| hsa-miR-497 | hsa-miR-373 | hsa-miR-188-3p | hsa-miR-302a# | hsa-miR-542-3p | hsa-miR-92b# | hsa-miR-1183 | |
| hsa-miR-543 | hsa-miR-335 | hsa-miR-671-3p | hsa-miR-30b# | hsa-miR-518d | hsa-miR-558 | hsa-miR-708# | |
| hsa-miR-505# | hsa-miR-339-3p | hsa-miR-21 | hsa-miR-425# | hsa-miR-455-3p | hsa-miR-580 | hsa-miR-27a# | |
| hsa-miR-548J | hsa-miR-34c | hsa-miR-654-3p | hsa-miR-21# | hsa-miR-576-5p | hsa-miR-130a# | hsa-miR-34a# | |
| hsa-miR-516-3p | hsa-miR-195 | hsa-miR-34a | hsa-miR-1200 | hsa-miR-802 | hsa-miR-100 | hsa-miR-204 | |
| hsa-miR-545# | hsa-miR-372 | hsa-miR-888 | hsa-miR-26a-2# | hsa-miR-92a-1# | hsa-1et-7d | hsa-miR-145 | |
| hsa-miR-590-5p | hsa-miR-149 | hsa-miR-652 | hsa-miR-578 | hsa-miR-767-3p | hsa-miR-210 | hsa-miR-154 | |
| hsa-miR-589 | hsa-miR-193a-5p | hsa-miR-871 | hsa-let-7i# | hsa-miR-1278 | hsa-miR-217 | hsa-miR-132 | |
| hsa-miR-625 | hsa-miR-136# | hsa-miR-1254 | hsa-1et-7g# | hsa-miR-888# | hsa-miR-22 | hsa-miR-140-3p | |
| hsa-miR-627 | hsa-miR-141# | hsa-miR-1255B | hsa-miR-222# | hsa-miR-93# | hsa-miR-103 | hsa-miR-15a | |
| hsa-miR-502-3p | hsa-miR-194# | hsa-miR-15b# | hsa-miR-448 | hsa-miR-609 | hsa-miR-365 | hsa-miR-186 | |
| hsa-miR-521 | hsa-miR-206 | hsa-miR-1226# | hsa-miR-376° | hsa-miR-620 | hsa-miR-370 | hsa-miR-887 | |
| hsa-miR-574-3p | hsa-miR-1274A | hsa-miR-124# | hsa-miR-431 | hsa-miR-585 | hsa-miR-328 | hsa-miR-744 | |
| hsa-miR-532 | hsa-miR-340# | hsa-miR-1225-3P | hsa-miR-29b | hsa-miR-1292 | hsa-miR-197 | hsa-miR-125a-3p | |
| hsa-miR-548b | hsa-miR-33a | hsa-miR-1228# | hsa-miR-454 | hsa-miR-548E | hsa-miR-338-3p | hsa-miR-653 | |

**Table 2. circulating miRNA and proteins associated with specific tumors.**

| **Tumor localization** | **Circulating miRNAs** | **Ref** | **Proteins and/or other markers (including age and gender)** | **Ref** |
|---|---|---|---|---|
| **Liver** | miR-21, miR-122, miR-223; miR-375+miR-25+1et-7f; miR-23b+miR-423+miR-375+miR-23a+miR-342-3p; | 22 | GALAD score, OPN, MDK, DKK-1, GPC3, α-1-Fucosidase, GP73; SCC Ag | 23 |
| | miR-122-5p+miR-100-5p+miR-125b-5p+miR-885-5p+miR-148a-3p | 24 | AFP, AFP-L3, DCP, GPC3, GP73, OPN, MDK, SCCA | 25 |
| | miR-21+miR-199a | 23 | cfDNA mutations+AFP+DCP | 26 |
| | miR-21+miR-199+miR-122 | 27 | AFP, AFP-L3, DCP, AFP-L3+DCP, AXL, AXL+AFP, TRX, TRX+AFP, GP73, GP73+AFP, GPC3, OPN, SCCA, ANXA2, ANXA2+AFP, suPAR, MDK | 28 |
| | miR-92a-3p+miR-107+miR-3126-5p+AFP | 29 | AFP+PIVKA-II+age+gender | 30 |
| | miR-29a+miR-29c+miR-133a+miR-143+miR-145+miR-192+miR-505 | 31 | | |
| | miR-122+1et-7; miR-122+miR-885-5p+miR-29b+AFP; miR-143+miR-215+AFP; miR-199a-3p, miR-210, miR-106b | 32 | | |
| **Lung** | miR-92a-3p+miR-30b-5p+miR-191-5p+miR-484+miR-328-3p+miR-30c-5p+miR-374a-5p+1et-7d-5p+miR-331-3p+miR-29a-3p+miR-148a-3p+miR-223-3p+miR-140-5p | 33 | CA125+CEA+CYFRA 21-1+Pro-SFTPB | 34 |
| | miR-19b+miR-25+miR-183 | 35 | CKAP4 | 36 |
| | miR-21+miR-339-5D | 37 | CRP, HGF | 38 |
| | miR-34b+miR-125b+miR-200b+miR-203+miR-205+miR-429 | 39 | | |
| | miR-339-3p+miR-425-3p+miR-532+miR-628-3p | 40 | | |
| | miR-574-5p+miR-1254 | 41 | | |
| | miR-1244 | 42 | | |
| **Ovary** | miR-320a-3p+miR-665+miR-3184-5p+miR-6717-5p+miR-4459+miR-6076+miR-3195+miR-1275+miR-3185+miR-4640-5p | 43 | CA125+AFP+HE4 | 3 |
| | miR-21+miR-141+miR-200a+miR-200b+miR-200c+miR-203+miR-205+miR-214 | 44 | CA125+SPINT1+TACSTD2+CLEC6A +ICOSLG +MSMB+PROK1 +CDH3+HE4+KRT19+FR-alpha | 20 |
| | miR-182+miR-200a+miR-200b+miR-200c | 45 | CA125+ CRP+ PZ+ LCAT | 21 |
| | miR-200a, miR-200b, miR-200c | 46 | | |
| **Pancreas** | miR-200a, miR-200b, miR-1290 | 22 | MUC5AC+CA19-9 | 47 |
| | | | | |
| | miR-20a+miR-21+miR-24+miR-25+miR-99a+miR-185+miR-191 | | | |
| | miR-1246+miR-4644+miR-3976+miR-4306+exosomal protein | 48 | TSP-2+CA19-9 | 49 |
| | miR-145+miR-150+miR-223+miR-636; miR-26b+miR-34a+miR-122+miR-126+miR145+miR-150+miR-223+miR-505+miR-636+miR-885-5p | 50 | Exosomal protein (CD44v6, Tspan8, EpCAM, MET, CD104)+miRNAs | 48 |
| | miR-486-5p and miR-938 | 51 | ctDNA mutations+CA19-9+CEA+HGF+OPN | 2 |
| | miR-21+miR-34a+miR-198+miR-217 | 52 | Exosomal GCP1 | 53 |
| **Stomach** | miR-16, miR-21 | 22 | CEA, CA19-9, CA72-4, CA125, CA24-2, CA50, PG, AFP | 54 |
| | | | | |
| | miR-1+miR-20a+miR-27a+miR-34+miR-423-5p | | | |
| | miR-16+miR-25+miR-92a+miR-451+miR-486-5p | 55 | IFNGR1, Notch-3, TNFRSF19L, GHR, SLAMF8, FR-beta, ITGA5, GAL-8, EphA1, EREG, FGF-12 | 56 |
| | miR-17-5p+ miR-21+ miR-106a+ miR-106b+1et-7a | 57 | CEACAM5, CA9, MSLN, CCL20, SCF, TGF-alpha,MMP-1,MMP-10, IGF-1, CDCP1, PPIA, DDAH-1, HMOX-1, FLI1, IL-7, ZBTB-17, APBB1IP, KAZALD-1, and ADAMTS-15 | 58 |
| | miR-21+miR-106b+miR-17+miR-18a+miR-20a | 59 | | |

| | | | | |
|---|---|---|---|---|
| AFP: alpha-Fetoprotein; OPN: Osteopontin; MDK: Midkine; GALAD: gender+age+ Alpha fetoprotein L3 (AFP-L3)+AFP+ Des-carboxy-prothrombin (DCP); DKK-1: Dickkopf-1; GPC3: Glypican-3; α-1-Fucosidase: Alpha-1-fucosidase; GP73: Golgi protein-73; SCC Ag: Squamous cell carcinoma antigen; AXL: protein AXL; TRX: Thioredoxin; ANXA2: Annexin A2; suPAR: soluble urokinase-type plasminogen activator receptor; PIVKA-II: Proteins Induced by Vitamin K Absence; CA125: Cancer Antigen 125; CEA: Carcinoembryonic Antigen; CYFRA 21-1: Cytokeratin 19 Fragment; Pro-SFTPB: pro-surfactant protein B; CKAP4: Cytoskeleton-associated protein 4; CRP: C-reactive protein; HGF: prolactin and hepatocyte growth factor; HE4: WAP four-disulfide core domain protein 2SPINT1: serine peptidase inhibitor Kunitz type 1; TACSTD2: Tumor-associated calcium signal transducer 2; CLEC6A: C-type lectin domain family 6 member A; ICOSLG: T-cell-specific cell surface receptor ligand; MSMB: Beta-microseminoprotein; PROK1: Prokineticin-1 precursor; CDH3: Cadherin-3; KRT19: Keratin, type I cytoskeletal 19; FR-alpha: Folate receptor alpha; PZ: Protein Z; LCAT: Lecithin cholesterol acyltransferase; MUC5AC: Mucin-5AC; CA19-9: Carbohydrate Antigen 19-9; TSP-2: Thrombospondin-2; CD44v6: cell-surface glycoprotein 44 v6; Tspan8: Tetraspanin-8; EpCAM: epithelial cellular adhesion molecule; MET: Hepatocyte growth factor receptor; CD104: cell-surface glycoprotein 104; GCP1: Germination-specific cysteine protease 1; CA72-4: carbohydrate antigens 72-4; CA24-2: carbohydrate antigens 24-2; CA50: carbohydrate antigens 50; PG: pepsinogen; IFNGR1: interferon gamma receptor 1; Notch-3: neurogenic locus notch homolog protein; TNFRSF19L: tumor necrosis factor receptor super family member 19L; GHR: growth hormone receptor; SLAMF8: signaling lymphocytic activation molecule family 8; FR-beta: folate receptor beta; ITGA5: integrin alpha 5; GAL-8: galectin-8; EphA1: erythropoietin-producing hepatocellular A1; EREG: epiregulin; FGF-12: fibroblast growth factor 12; CEACAM5: Carcinoembryonic antigen-related cell adhesion molecule 5; CA9: Carbonic anhydrase 9; MSLN: Mesothelin; CCL20: Chemokine ligand 20; SCF: Skp1, Cullins, F-box proteins; TGF-alpha: Transforming growth factor alpha; MMP-1: Matrix metalloproteinase-1; MMP-10: Matrix metalloproteinase-10; IGF-1: Insulin-like growth factor 1; CDCP1: CUB domain-containing protein 1; PPIA: Peptidylprolyl isomerase A; DDAH-1: N(G),N(G)-dimethylarginine dimethylaminohydrolase 1; HMOX-1: Heme oxygenase 1; FLI1: Friend leukemia integration 1 transcription factor; IL-7: Interleukin 7; ZBTB-17: Zinc Finger And BTB Domain Containing 17; APBB1IP: Amyloid Beta Precursor Protein Binding Family B Member 1 Interacting Protein; KAZALD-1: Kazal Type Serine Peptidase Inhibitor Domain; ADAMTS-15: disintegrin and metalloproteinase with thrombospondin motifs 15. | | | | |

**Table 3. Performance of the test of the multi-analytical model.**

| **Tumor localization** | **Circulating miRNAs+proteins and/or other personal features** | **N (Cases)** | **N (Controls)** | **AUC** | **Sensitivity %** | **Specificity %** |
|---|---|---|---|---|---|---|
| **Liver** | miR-122, miR-21, miR-223, miR-26a, miR-27a, miR-801, miR-1972, miR-193a-5p, miR-214-3p, miR-365a-3p, miR-92-3p, miR-107, miR-3126-5p, miR-29a, miR-29c, miR-133a, miR-143, miR-145, miR-192, miR-505+AFP, PIVKA-II, AFP-L3, DCP+age+gender | 2577 | 1330 | 0.89 | 81.31 | 87.45 |
| **Lung** | miR-92a-3p, miR-30b-5p, miR-191-5p, miR-484, miR-328-3p miR-30c-5p miR-374a-5p, 1et-7d-5p, miR-331-3p, miR-29a-3p, miR-148a-3p, miR-223-3p, miR-140-5p, miR-19b, miR-25, miR-183, miR-21, miR-339-5p, miR-429, miR-205, miR-200b, miR-203, miR-125b, miR-34b, miR-532, miR-628-3p, miR-425-3p, miR-1254, miR-574-5p, miR-182-5p, miR-10a-5p, miR-301b, miR-1244, miR-301a-3p, miR-135b-5p, miR-224-5p, miR-21-5p+CKAP4, CRP, Prolactin, HGF, CEA | 2775 | 2085 | 0.89 | 85.46 | 88.11 |
| **Ovary** | miR-320a-3p, miR-665, miR-3184-5p, miR-6717-5p, miR-4459, miR-6076, miR-3195, miR-1275, miR-3185, miR-4640-5p, miR-200b, miR-200c, miR-191-5p, miR-423-3p, miR-320b, miR-141-3p, miR-625-3p, miR-145-3p, miR-92b-5p, miR-320c, miR-320a-3p, miR-320d+CA125, SPINT1, TACSTD2, CLEC6A, ICOSLG, MSMB, PROK1, CDH3, HE4, KRT19, FR-alpha, CRP, PZ, LCAT+age | 790 | 3652 | 0.91 | 88.93 | 83.35 |
| **Pancreas** | miR-1246, miR-4644, miR-3976, miR-4306, miR-145, miR-150, miR-26b, miR-34a, miR-122, miR-223, miR-505, miR-636, miR-885-5p+CD44v6, Tspan8, EpCAM, MET, CD104, THBS2, CA19-9 | 1379 | 578 | 0.96 | 90.00 | 88.25 |
| **Stomach** | miR-16, miR-25, miR-92a, miR-451, miR-486-5p, miR-17-5p, miR-21, miR-106a, miR-106b+CEACAM5, CA9, MSLN, CCL20, SCF, TGF-alpha,MMP-1,MMP-10, IGF-1, CDCP1, PPIA, DDAH-1, HMOX-1, FLI1, IL-7, ZBTB-17, APBB1IP, KAZALD-1, ADAMTS-15 | 268 | 186 | 0.96 | 88.78 | 92.70 |

### Bibliography

1. Siravegna, G., Marsoni, S., Siena, S. & Bardelli, A. Integrating liquid biopsies into the management of cancer. Nature Reviews Clinical Oncology (2017). doi: 10.1038/nrclinonc.2017.14
2. Cohen, J. D. et al. Combined circulating tumor DNA and protein biomarker-based liquid biopsy for the earlier detection of pancreatic cancers. Proc. Natl. Acad. Sci. U. S. A. (2017). doi:10.1073/pnas.1704961114
3. Cohen, J. D. et al. Detection and localization of surgically resectable cancers with a multi-analyte blood test. Science (80-.). (2018). doi:10.1126/science.aar3247
4. Sheridan, C. Investors keep the faith in cancer liquid biopsies. Nat. Biotechnol. (2019). doi:10.1038/d41587-019-00022-7
5. Vogelstein, B. & Kinzler, K. W. Digital PCR. Proc. Natl. Acad. Sci. U. S. A. (1999). doi:10.1073/pnas.96.16.9236
6. Knebel, F. H. et al. Sequential liquid biopsies reveal dynamic alterations of EGFR driver mutations and indicate EGFR amplification as a new mechanism of resistance to osimertinib in NSCLC. Lung Cancer (2017). doi:10.1016/j.lungcan.2017.04.004
7. Carpinetti, P. et al. The use of personalized biomarkers and liquid biopsies to monitor treatment response and disease recurrence in locally advanced rectal cancer after neoadjuvant chemoradiation. Oncotarget (2015). doi:10.18632/oncotarget.5256
8. Nakamura, K. et al. Clinical relevance of circulating cell-free microRNAs in ovarian cancer. Mol. Cancer 15, 48 (2016).
9. Schwarzenbach, H., Nishida, N., Calin, G. A. & Pantel, K. Clinical relevance of circulating cell-free microRNAs in cancer. Nature Reviews Clinical Oncology (2014). doi:10.103 8/nrclinonc.2014.5
10. Miotto, E. et al. Quantification of circulating miRNAs by droplet digital PCR: Comparison of EvaGreen- and TaqMan-based chemistries. Cancer Epidemiol. Biomarkers Prev. (2014). doi:10.1158/1055-9965.EPI-14-0503
11. Zhao, G. et al. Droplet digital PCR-based circulating microRNA detection serve as a promising diagnostic method for gastric cancer. BMC Cancer (2018). doi:10.1186/s12885-018-4601-5
12. Wang, C. et al. Droplet digital PCR improves urinary exosomal miRNA detection compared to real-time PCR. Clin. Biochem. (2019). doi:10.1016/j.clinbiochem.2019.03.008
13. Tumor Markers - National Cancer Institute. Available at: https://www.cancer.gov/about-cancer/diagnosis-staging/diagnosis/tumor-markers-fact-sheet. (Accessed: 8th November 2019)
14. Hazelton, W. D. & Luebeck, E. G. Biomarker-based early cancer detection: Is it achievable? Science Translational Medicine (2011). doi:10.1126/scitranslmed.3003272
15. Bettegowda, C. et al. Detection of circulating tumor DNA in early- and late-stage human malignancies. Sci. Transl. Med. (2014). doi:10.1126/scitranslmed.3007094
16. McDonald, J. S., Milosevic, D., Reddi, H. V., Grebe, S. K. & Algeciras-Schimnich, A. Analysis of circulating microRNA: Preanalytical and analytical challenges. Clin. Chem. (2011). doi:10.1373/clinchem.2010.157198
17. Faraldi, M. et al. Normalization strategies differently affect circulating miRNA profile associated with the training status. Sci. Rep. (2019). doi:10.1038/s41598-019-38505-x
18. Mari, R. et al. Liquid Biopsies for Ovarian Carcinoma: How Blood Tests May Improve the Clinical Management of a Deadly Disease. Cancers (Basel). (2019). doi:10.3390/cancers11060774
19. TaqMan® OpenArray® Human MicroRNA Panel. Available at: https://www.thermofisher.com/order/catalog/product/4461104?SID=srch-srp-4461104#/4461104?SID=srch-srp-4461104. (Accessed: 21st November 2019)
20. Enroth, S. et al. High throughput proteomics identifies a high-accuracy 11 plasma protein biomarker signature for ovarian cancer. Commun. Biol. (2019). doi:10.1038/s42003-019-0464-9
21. Russell, M. R. et al. Diagnosis of epithelial ovarian cancer using a combined protein biomarker panel. Br. J. Cancer (2019). doi:10.1038/s41416-019-0544-0
22. Shigeyasu, K., Toden, S., Zumwalt, T. J., Okugawa, Y. & Goel, A. Emerging role of microRNAs as liquid biopsy biomarkers in gastrointestinal cancers. Clinical Cancer Research (2017). doi:10.1158/1078-0432.CCR-16-1676
23. Sengupta, S. & Parikh, N. D. Biomarker development for hepatocellular carcinoma early detection: current and future perspectives. Hepatic Oncol. (2017). doi:10.2217/hep-2017-0019
24. Jin, Y. et al. Circulating microRNAs as Potential Diagnostic and Prognostic Biomarkers in Hepatocellular Carcinoma. Sci. Rep. (2019). doi:10.1038/s41598-019-46872-8
25. Liu, X. N. et al. Multiple 'Omics' data-based biomarker screening for hepatocellular carcinoma diagnosis. World Journal of Gastroenterology (2019). doi:10.3748/wjg.v25.i30.4199
26. Qu, C. et al. Detection of early-stage hepatocellular carcinoma in asymptomatic HBsAg-seropositive individuals by liquid biopsy. Proc. Natl. Acad. Sci. U. S. A. (2019). doi:10.1073/pnas.1819799116
27. Ding, Y., Yan, J. L., Fang, A. N., Zhou, W. F. & Huang, L. Circulating miRNAs as novel diagnostic biomarkers in hepatocellular carcinoma detection: A meta-analysis based on 24 articles. Oncotarget (2017). doi:10.18632/oncotarget.18949
28. Chia, T. S., Wong, K. F. & Luk, J. M. Molecular diagnosis of hepatocellular carcinoma: trends in biomarkers combination to enhance early cancer detection. (2019).
29. Zhang, Y. et al. Serum microRNA panel for early diagnosis of the onset of hepatocellular carcinoma. Med. (United States) (2017). doi: 10. 1097/MD.0000000000005642
30. Hemken, P. M. et al. Validation of a novel model for the early detection of hepatocellular carcinoma. in Clinical Proteomics (2019). doi:10.1186/s12014-018-9222-0
31. Lin, X. J. et al. A serum microRNA classifier for early detection of hepatocellular carcinoma: A multicentre, retrospective, longitudinal biomarker identification study with a nested case-control study. Lancet Oncol. (2015). doi:10.1016/S1470-2045(15)00048-0
32. Pezzuto, F., Buonaguro, L., Buonaguro, F. M. & Tornesello, M. L. The role of circulating free DNA and microRNA in non-invasive diagnosis of HBV- and HCV-related hepatocellular carcinoma. International Journal of Molecular Sciences (2018). doi:10.3390/ijms19041007
33. Montani, F. et al. MiR-test: A blood test for lung cancer early detection. J. Natl. Cancer Inst. (2015). doi:10.1093/jnci/djv063
34. Guida, F. et al. Assessment of lung cancer risk on the basis of a biomarker panel of circulating proteins. JAMA Oncol. 4, e182078-e182078 (2018).
35. Zaporozhchenko, I. A. et al. Plasma miR-19b and miR-183 as potential biomarkers of lung cancer. PLoS One (2016). doi:10.1371/journal.pone.0165261
36. Yanagita, K. et al. Cytoskeleton-Associated Protein 4 Is a Novel Serodiagnostic Marker for Lung Cancer. Am. J. Pathol. (2018). doi:10.1016/j.ajpath.2018.03.007
37. Sun, Y., Mei, H., Xu, C., Tang, H. & Wei, W. Circulating microRNA-339-5p and-21 in plasma as an early detection predictors of lung adenocarcinoma. Pathol. Pract. 214, 119-125 (2018).
38. Ma, S. et al. Multiplexed Serum Biomarkers for the Detection of Lung Cancer. EBioMedicine (2016). doi:10.1016/j.ebiom.2016.08.018
39. Halvorsen, A. R. et al. A unique set of 6 circulating microRNAs for early detection of non-small cell lung cancer. Oncotarget (2016). doi:10.18632/oncotarget.9363
40. Wang, Y. et al. Identification of a three-miRNA signature as a blood-borne diagnostic marker for early diagnosis of lung adenocarcinoma. Oncotarget (2016). doi:10.18632/oncotarget.8429
41. Foss, K. M. et al. MiR-1254 and miR-574-5p: Serum-based microRNA biomarkers for early-stage non-small cell lung cancer. J. Thorac. Oncol. (2011). doi:10.1097/JTO.0b013e318208c785
42. Wang, W. et al. Identification of miRNAs as non-invasive biomarkers for early diagnosis of lung cancers. Tumor Biol. (2016). doi:10.1007/s13277-016-5442-y
43. Yokoi, A. et al. Integrated extracellular microRNA profiling for ovarian cancer screening. Nat. Commun. (2018). doi:10.1038/s41467-018-06434-4
44. Taylor, D. D. & Gercel-Taylor, C. MicroRNA signatures of tumor-derived exosomes as diagnostic biomarkers of ovarian cancer. Gynecol. Oncol. (2008). doi:10.1016/j.ygyno.2008.04.033
45. Kan, C. W. S. et al. Elevated levels of circulating microRNA-200 family members correlate with serous epithelial ovarian cancer. BMC Cancer (2012). doi:10.1186/1471-2407-12-627
46. Zuberi, M. et al. Expression of serum miR-200a, miR-200b, and miR-200c as candidate biomarkers in epithelial ovarian cancer and their association with clinicopathological features. Clin. Transl. Oncol. (2015). doi:10.1007/s12094-015-1303-1
47. Kaur, S. et al. A Combination of MUC5AC and CA19-9 Improves the Diagnosis of Pancreatic Cancer: A Multicenter Study. Am. J. Gastroenterol. (2017). doi:10.1038/ajg.2016.482
48. Madhavan, B. et al. Combined evaluation of a panel of protein and miRNA serum-exosome biomarkers for pancreatic cancer diagnosis increases sensitivity and specificity. Int. J. Cancer (2015). doi:10.1002/ijc.29324
49. Kim, J. et al. Detection of early pancreatic ductal adenocarcinoma with thrombospondin-2 & CA19-9 blood markers. Sci. Transl. Med. (2017). doi:10.1126/scitranslmed.aah5583
50. Schultz, N. A. et al. MicroRNA biomarkers in whole blood for detection of pancreatic cancer. JAMA - J. Am. Med. Assoc. (2014). doi:10.1001/jama.2013.284664
51. Le Large, T. Y. S. et al. Circulating microRNAs as diagnostic biomarkers for pancreatic cancer. Expert Rev. Mol. Diagn. (2015). doi:10.1586/14737159.2015.1112273
52. Vychytilova-Faltejskova, P. et al. MiR-21, miR-34a, miR-198 and miR-217 as diagnostic and prognostic biomarkers for chronic pancreatitis and pancreatic ductal adenocarcinoma. Diagn. Pathol. (2015). doi:10.1186/s13000-015-0272-6
53. Herreros-Villanueva, M. & Bujanda, L. Glypican-1 in exosomes as biomarker for early detection of pancreatic cancer. Annals of Translational Medicine (2016). doi:10.3978/j.issn.2305-5839.2015.10.39
54. Tsai, M. M. et al. Potential diagnostic, prognostic and therapeutic targets of micrornas in human gastric cancer. International Journal of Molecular Sciences (2016). doi:10.3390/ijms17060945
55. Zhu, C. et al. A five-microRNA panel in plasma was identified as potential biomarker for early detection of gastric cancer. Br. J. Cancer (2014). doi:10.1038/bjc.2014.119
56. Wu, D. et al. Serum biomarker panels for the diagnosis of gastric cancer. Cancer Med. (2019). doi:10.1002/cam4.2055
57. Tsujiura, M. et al. Circulating microRNAs in plasma of patients with gastric cancers. Br. J. Cancer (2010). doi:10.1038/sj.bjc.6605608
58. Shen, Q. et al. A targeted proteomics approach reveals a serum protein signature as diagnostic biomarker for resectable gastric cancer. EBioMedicine (2019). doi: 10.1016/j.ebiom.2019.05.044
59. Wang, J. L. et al. Candidate microRNA Biomarkers in Human Gastric Cancer: A Systematic Review and Validation Study. PLoS One (2013). doi:10.1371/journal.pone.0073683

## Claims

1. Non-invasive diagnosis method for the early determination of cancer by means of liquid biopsy, said method allowing the identification, in a peripheral blood sample previously drawn from the patient, of the expression profile of circulating miRNA and of serum proteins released in the blood circulation by solid tumors, said method also considering markers such as age and sex and allowing the early analysis of a pathological state in initial phase.

2. Non-invasive diagnosis method for the early determination of cancer by means of liquid biopsy according to the preceding claim **wherein** ddPCR is employed as miRNA quantification technique.

3. Non-invasive diagnosis method for the early determination of cancer by means of liquid biopsy according to the preceding claims sequentially comprising the following passages:
- Purification of miRNA and proteins from serum;
- Reverse transcription of the total miRNA;
- Quantification of miRNA by means of ddPCR;
- Quantification of the serum proteins;
- Statistical analysis for the determination of the diagnostic indices.

4. Non-invasive diagnosis method for the early determination of cancer by means of liquid biopsy according to the preceding claim **wherein** the statistical analysis provides that the diagnostic indices are generated by using an algorithm developed based on Fisher's linear discriminant analysis, said statistical analysis providing that an index ≥ 0 indicates a tumor state and an index ≤ 0 indicates the absence of tumor.

5. Non-invasive diagnosis method for the early determination of cancer by means of liquid biopsy according to the preceding claim **wherein** the miRNA identified for the detection of a tumor state were selected from among the miRNA listed in the following table:
| | | | | | | | |
|---|---|---|---|---|---|---|---|
| hsa-miR-1272 | hsa-miR-658 | hsa-miR-486 | hsa-miR-518b | hsa-miR-193b | hsa-miR-891a | hsa-miR-409-5p | hsa-miR-556-3p |
| hsa-miR-146a# | hsa-miR-648 | hsa-miR-26a | hsa-miR-503 | hsa-miR-199b | hsa-miR-1260 | hsa-miR-424 | hsa-miR-548a |
| hsa-miR-1274B | hsa-miR-668 | hsa-miR-220c | hsa-miR-509-3-5p | hsa-miR-195# | hsa-miR-190b | hsa-miR-30b | hsa-miR-587 |
| hsa-miR-1285 | hsa-miR-663B | hsa-miR-10a | hsa-miR-502 | hsa-miR-34b | hsa-miR-1227 | hsa-miR-29a | hsa-miR-1286 |
| hsa-miR-1293 | hsa-miR-720 | hsa-miR-489 | hsa-miR-516a-5p | hsa-miR-337-3p | hsa-miR-1256 | hsa-miR-485-3p | hsa-miR-596 |
| hsa-miR-1298 | hsa-miR-766 | hsa-miR-222 | hsa-miR-508 | hsa-miR-577 | hsa-miR-1250 | hsa-miR-484 | hsa-miR-621 |
| hsa-miR-200a | hsa-miR-640 | hsa-miR-105 | hsa-miR-200c | hsa-miR-33a# | hsa-miR-1205 | hsa-miR-380-3p | hsa-miR-605 |
| hsa-miR-374 | hsa-miR-875-5p | hsa-miR-221 | hsa-miR-525 | hsa-miR-645 | hsa-miR-744# | hsa-miR-299-3p | hsa-miR-548M |
| hsa-miR-371-3p | hsa-miR-133a | hsa-miR-770-5p | hsa-miR-524-5p | hsa-miR-363# | hsa-miR-638 | hsa-miR-28 | hsa-miR-541# |
| hsa-miR-325 | hsa-miR-152 | hsa-miR-29b-2# | hsa-miR-520g | hsa-miR-16-1# | hsa-miR-661 | hsa-miR-320 | hsa-miR-520c-3p |
| hsa-miR-362 | hsa-miR-551b | hsa-miR-30c-1# | hsa-miR-518e | hsa-miR-138-2# | hsa-miR-650 | hsa-miR-376c | hsa-miR-519b-3p |
| hsa-miR-516b | hsa-miR-185 | hsa-miR-432# | hsa-miR-520f | hsa-miR-148b# | hsa-miR-7-2# | hsa-miR-423-5p | hsa-miR-548K |
| hsa-miR-19b | hsa-miR-191 | hsa-miR-32# | hsa-miR-301b | hsa-miR-569 | hsa-miR-639 | hsa-miR-422a | hsa-miR-548H |
| hsa-miR-331 | hsa-miR-133b | hsa-miR-454# | hsa-miR-941 | hsa-miR-144# | hsa-miR-146a | hsa-miR-377 | hsa-miR-552 |
| hsa-miR-136 | hsa-miR-190 | hsa-miR-30d | hsa-miR-588 | hsa-miR-1264 | hsa-miR-148b | hsa-miR-378 | hsa-miR-582-3p |
| hsa-miR-362-3p | hsa-miR-885-3p | hsa-miR-1180 | hsa-miR-513C | hsa-miR-1283 | hsa-miR-127 | hsa-miR-374a# | hsa-miR-23a |
| hsa-miR-339-5p | hsa-miR-873 | hsa-1et-7b# | hsa-miR-603 | hsa-miR-220 | hsa-miR-181a | hsa-miR-424# | hsa-miR-9 |
| hsa-miR-330-5p | hsa-miR-122 | hsa-miR-26a-1# | hsa-miR-937 | hsa-miR-224 | hsa-miR-139-5p | hsa-miR-409-3p | hsa-miR-562 |
| hsa-miR-519d | hsa-miR-876-5p | hsa-miR-223# | hsa-miR-617 | hsa-miR-361 | hsa-miR-142-5p | hsa-miR-29a# | hsa-miR-551a |
| hsa-miR-19a | hsa-miR-886-3p | hsa-miR-24-1# | hsa-miR-635 | hsa-miR-212 | hsa-miR-127-5p | hsa-miR-30d# | hsa-miR-572 |
| hsa-miR-193a-3p | hsa-miR-889 | hsa-1et-7c# | hsa-miR-614 | hsa-miR-192 | hsa-miR-130b | hsa-miR-30e-3p | hsa-miR-566 |
| hsa-miR-194 | hsa-miR-92a | hsa-miR-513-5p | hsa-miR-623 | hsa-1et-7c | hsa-miR-642 | hsa-1et-7f-2# | hsa-miR-575 |
| hsa-miR-144 | hsa-miR-122# | hsa-miR-382 | hsa-miR-518f# | hsa-miR-342-5p | hsa-miR-655 | hsa-miR-106b# | hsa-miR-96# |
| hsa-miR-149# | hsa-miR-1825 | hsa-miR-449b | hsa-miR-524 | hsa-miR-20b | hsa-miR-99a | hsa-miR-23b# | hsa-miR-99a# |
| hsa-miR-1294 | hsa-miR-641 | hsa-miR-141 | hsa-miR-626 | hsa-miR-331-5p | hsa-miR-99b | hsa-miR-221# | hsa-miR-556-3p |
| hsa-miR-148a# | hsa-miR-1255A | hsa-miR-32 | hsa-miR-548G | hsa-miR-33b | hsa-miR-651 | hsa-miR-1179 | hsa-miR-548a |
| hsa-miR-942 | hsa-miR-649 | hsa-miR-298 | hsa-miR-513B | hsa-miR-369-3p | hsa-miR-885-5p | hsa-miR-27b# | hsa-miR-587 |
| hsa-miR-1302 | hsa-miR-1224-3P | hsa-miR-486-3p | hsa-miR-548N | hsa-miR-369-5p | hsa-miR-636 | hsa-miR-10a# | hsa-miR-1286 |
| hsa-miR-1291 | hsa-miR-631 | hsa-miR-29c | hsa-miR-518a-3p | hsa-miR-208 | hsa-miR-891b | hsa-miR-453 | hsa-miR-596 |
| hsa-miR-16-2# | hsa-miR-924 | hsa-miR-125a-5p | hsa-miR-629 | hsa-miR-542-5p | hsa-miR-1233 | hsa-miR-485-5p | hsa-miR-621 |
| hsa-miR-15a# | hsa-miR-92a-2# | hsa-miR-10b | hsa-miR-598 | hsa-miR-20a# | hsa-miR-1204 | hsa-miR-449 | hsa-miR-605 |
| hsa-miR-1289 | hsa-miR-935 | hsa-miR-301 | hsa-miR-597 | hsa-miR-19a# | hsa-miR-126# | hsa-miR-302b | hsa-miR-548M |
| hsa-miR-1253 | hsa-miR-944 | hsa-miR-181c | hsa-miR-615-5p | hsa-miR-135b# | hsa-miR-1247 | hsa-miR-411 | hsa-miR-541# |
| hsa-miR-1303 | hsa-miR-938 | hsa-miR-491-3p | hsa-miR-624 | hsa-miR-145# | hsa-miR-1263 | hsa-miR-324-5p | hsa-miR-520c-3p |
| hsa-miR-1203 | hsa-miR-892b | hsa-miR-219-2-3p | hsa-miR-628-5p | hsa-miR-1290 | hsa-miR-1249 | hsa-miR-30c | hsa-miR-519b-3p |
| hsa-miR-1282 | hsa-miR-767-5p | hsa-miR-215 | hsa-miR-520e | hsa-miR-361-3p | hsa-miR-1206 | hsa-miR-299-5p | hsa-miR-548K |
| hsa-miR-1257 | hsa-1et-7a | hsa-miR-27b | hsa-miR-450b-5p | hsa-miR-1296 | hsa-miR-125b-2# | hsa-miR-214 | hsa-miR-548H |
| hsa-miR-186# | hsa-miR-211 | hsa-miR-380-5p | hsa-miR-570 | hsa-miR-1305 | hsa-miR-647 | hsa-miR-492 | hsa-miR-552 |
| hsa-miR-493 | hsa-miR-579 | hsa-miR-25# | hsa-miR-544 | hsa-miR-1276 | hsa-miR-335# | hsa-miR-483-5p | hsa-miR-582-3p |
| hsa-miR-501-3p | hsa-miR-556-5p | hsa-miR-320B | hsa-miR-548a-5p | hsa-miR-1826 | hsa-miR-662 | hsa-1et-7f | hsa-miR-23a |
| hsa-miR-512-5p | hsa-miR-758 | hsa-miR-106a# | hsa-miR-548d | hsa-miR-1304 | hsa-miR-659 | hsa-1et-7e | hsa-miR-9 |
| hsa-miR-507 | hsa-miR-890 | hsa-miR-29b-1# | hsa-miR-26b | hsa-miR-1259 | hsa-miR-675 | hsa-miR-490 | hsa-miR-562 |
| hsa-miR-505 | hsa-miR-205 | hsa-miR-214# | hsa-miR-630 | hsa-miR-132# | hsa-miR-934 | hsa-miR-30a-5p | hsa-miR-551a |
| hsa-miR-518c | hsa-miR-1208 | hsa-miR-1184 | hsa-miR-586 | hsa-miR-1267 | hsa-miR-765 | hsa-miR-376a# | hsa-miR-572 |
| hsa-miR-511 | hsa-miR-564 | hsa-1et-7f-1# | hsa-miR-590-3P | hsa-miR-346 | hsa-miR-769-5p | hsa-miR-302b# | hsa-miR-566 |
| hsa-miR-518a-5p | hsa-miR-581 | hsa-miR-1197 | hsa-miR-10b# | hsa-miR-340 | hsa-miR-182 | hsa-miR-302d | hsa-miR-575 |
| hsa-miR-520d-5p | hsa-miR-555 | hsa-miR-202# | hsa-miR-517# | hsa-miR-345 | hsa-miR-150 | hsa-miR-30c-2# | hsa-miR-96# |
| hsa-miR-155 | hsa-miR-563 | hsa-miR-218-1# | hsa-miR-592 | hsa-miR-509-5p | hsa-miR-129 | hsa-miR-31# | hsa-miR-99a# |
| hsa-miR-541 | hsa-miR-583 | hsa-miR-196a# | hsa-miR-613 | hsa-miR-198 | hsa-miR-18b | hsa-miR-431# | hsa-miR-556-3p |
| hsa-miR-523 | hsa-miR-573 | hsa-miR-1178 | hsa-miR-600 | hsa-miR-203 | hsa-miR-15b | hsa-miR-100# | hsa-miR-548a |
| hsa-miR-921 | hsa-miR-554 | hsa-miR-143# | hsa-miR-5481 | hsa-miR-200b | hsa-miR-184 | hsa-miR-213 | hsa-miR-587 |
| hsa-miR-933 | hsa-miR-296 | hsa-miR-367# | hsa-miR-548L | hsa-miR-202 | hsa-miR-18a | hsa-miR-1182 | hsa-miR-1286 |
| hsa-miR-1284 | hsa-miR-1 | hsa-miR-338-5P | hsa-miR-520D-3P | hsa-miR-508-5p | hsa-miR-875-3p | hsa-1et-7a# | hsa-miR-596 |
| hsa-miR-411# | hsa-miR-216b | hsa-miR-135a | hsa-miR-500 | hsa-miR-139-3p | hsa-miR-892a | hsa-miR-193b# | hsa-miR-621 |
| hsa-miR-920 | hsa-miR-223 | hsa-miR-146b-3p | hsa-miR-519e# | hsa-miR-517a | hsa-miR-146b | hsa-miR-22# | hsa-miR-605 |
| hsa-miR-936 | hsa-miR-329 | hsa-miR-147b | hsa-miR-550 | hsa-miR-520a | hsa-miR-874 | hsa-miR-24-2# | hsa-miR-548M |
| hsa-miR-616 | hsa-miR-24 | hsa-miR-128a | hsa-miR-561 | hsa-miR-199a | hsa-miR-876-3p | hsa-miR-381 | hsa-miR-541# |
| hsa-miR-591 | hsa-miR-101 | hsa-miR-130a | hsa-miR-324-3p | hsa-miR-526b | hsa-miR-886-5p | hsa-miR-376b | hsa-miR-520c-3p |
| hsa-miR-599 | hsa-miR-515-5p | hsa-miR-143 | hsa-miR-95 | hsa-miR-196b | hsa-miR-337-5p | hsa-miR-296-3p | hsa-miR-519b-3p |
| hsa-miR-608 | hsa-miR-20a | hsa-miR-654 | hsa-miR-487b | hsa-miR-519e | hsa-miR-1245 | hsa-miR-412 | hsa-miR-548K |
| hsa-miR-501 | hsa-miR-363 | hsa-miR-106b | hsa-miR-557 | hsa-miR-191# | hsa-miR-637 | hsa-miR-429 | hsa-miR-548H |
| hsa-miR-518d-5p | hsa-miR-148a | hsa-miR-872 | hsa-miR-548P | hsa-miR-154# | hsa-miR-1244 | hsa-miR-450a | hsa-miR-552 |
| hsa-miR-506 | hsa-miR-330 | hsa-miR-125b | hsa-miR-601 | hsa-miR-151-5P | hsa-miR-1262 | hsa-miR-455 | hsa-miR-582-3p |
| hsa-miR-512-3p | hsa-miR-367 | hsa-miR-660 | hsa-miR-553 | hsa-miR-1300 | hsa-miR-551b# | hsa-miR-302c | hsa-miR-23a |
| hsa-miR-618 | hsa-miR-200a# | hsa-miR-708 | hsa-miR-571 | hsa-miR-151-3p | hsa-miR-1238 | hsa-miR-219 | hsa-miR-9 |
| hsa-miR-517b | hsa-miR-200b# | hsa-miR-483-3p | hsa-miR-518e# | hsa-miR-1265 | hsa-miR-656 | hsa-miR-23b | hsa-miR-562 |
| hsa-miR-510 | hsa-miR-192# | hsa-miR-18b# | hsa-miR-126 | hsa-miR-181a-2# | hsa-miR-9# | hsa-miR-674 | hsa-miR-551a |
| hsa-miR-539 | hsa-miR-200c# | hsa-miR-125b-1# | hsa-miR-433 | hsa-miR-182# | hsa-miR-644 | hsa-miR-17 | hsa-miR-572 |
| hsa-miR-532-3p | hsa-miR-20b# | hsa-miR-1248 | hsa-miR-452 | hsa-miR-1275 | hsa-miR-922 | hsa-1et-7g | hsa-miR-566 |
| hsa-miR-519c | hsa-miR-664 | hsa-miR-155# | hsa-miR-425-5p | hsa-miR-183# | hsa-miR-939 | hsa-miR-216a | hsa-miR-575 |
| hsa-miR-545 | hsa-miR-99b# | hsa-miR-1252 | hsa-miR-302° | hsa-miR-1270 | hsa-miR-549 | hsa-miR-219-1-3p | hsa-miR-96# |
| hsa-miR-520b | hsa-miR-17# | hsa-miR-633 | hsa-miR-375 | hsa-miR-1236 | hsa-miR-769-3p | hsa-miR-672 | hsa-miR-99a# |
| hsa-miR-519a | hsa-miR-1288 | hsa-miR-646 | hsa-miR-323-3p | hsa-miR-181c# | hsa-miR-943 | hsa-miR-302c# | hsa-miR-556-3p |
| hsa-miR-522 | hsa-miR-1271 | hsa-miR-665 | hsa-miR-410 | hsa-miR-595 | hsa-miR-548c | hsa-miR-218-2# | hsa-miR-548a |
| hsa-miR-520a# | hsa-miR-1269 | hsa-miR-657 | hsa-miR-487° | hsa-miR-18a# | hsa-miR-582-5p | hsa-miR-432 | hsa-miR-587 |
| hsa-miR-497# | hsa-miR-129# | hsa-miR-634 | hsa-miR-28-3p | hsa-miR-1243 | hsa-miR-576-3p | hsa-miR-1201 | hsa-miR-1286 |
| hsa-miR-593 | hsa-miR-1301 | hsa-miR-643 | hsa-miR-383 | hsa-1et-7b | hsa-miR-548d-5p | hsa-miR-302d# | hsa-miR-596 |
| hsa-miR-607 | hsa-miR-130b# | hsa-miR-142-3p | hsa-miR-107 | hsa-miR-499-3p | hsa-miR-326 | hsa-1et-7e# | |
| hsa-miR-606 | hsa-miR-218 | hsa-miR-138 | hsa-miR-384 | hsa-miR-494 | hsa-miR-208b | hsa-miR-488 | |
| hsa-miR-622 | hsa-miR-25 | hsa-miR-16 | hsa-miR-450b-3p | hsa-miR-504 | hsa-miR-98 | hsa-miR-23a# | |
| hsa-miR-518c# | hsa-miR-106a | hsa-miR-548b-5p | hsa-miR-374b# | hsa-miR-515-3p | hsa-miR-559 | hsa-miR-105# | |
| hsa-miR-628-3p | hsa-miR-27a | hsa-miR-147 | hsa-miR-377# | hsa-miR-517c | hsa-miR-584 | hsa-miR-26b# | |
| hsa-miR-625# | hsa-miR-199a-3p | hsa-miR-135b | hsa-miR-452# | hsa-miR-525-3p | hsa-miR-30a-3p | hsa-miR-101# | |
| hsa-miR-520h | hsa-miR-220b | hsa-miR-183 | hsa-miR-567 | hsa-miR-518f | hsa-miR-1324 | hsa-miR-19b-1# | |
| hsa-miR-497 | hsa-miR-373 | hsa-miR-188-3p | hsa-miR-302a# | hsa-miR-542-3p | hsa-miR-92b# | hsa-miR-1183 | |
| hsa-miR-543 | hsa-miR-335 | hsa-miR-671-3p | hsa-miR-30b# | hsa-miR-518d | hsa-miR-558 | hsa-miR-708# | |
| hsa-miR-505# | hsa-miR-339-3p | hsa-miR-21 | hsa-miR-425# | hsa-miR-455-3p | hsa-miR-580 | hsa-miR-27a# | |
| hsa-miR-548J | hsa-miR-34c | hsa-miR-654-3p | hsa-miR-21# | hsa-miR-576-5p | hsa-miR-130a# | hsa-miR-34a# | |
| hsa-miR-516-3p | hsa-miR-195 | hsa-miR-34a | hsa-miR-1200 | hsa-miR-802 | hsa-miR-100 | hsa-miR-204 | |
| hsa-miR-545# | hsa-miR-372 | hsa-miR-888 | hsa-miR-26a-2# | hsa-miR-92a-1# | hsa-1et-7d | hsa-miR-145 | |
| hsa-miR-590-5p | hsa-miR-149 | hsa-miR-652 | hsa-miR-578 | hsa-miR-767-3p | hsa-miR-210 | hsa-miR-154 | |
| hsa-miR-589 | hsa-miR-193a-5p | hsa-miR-871 | hsa-1et-7i# | hsa-miR-1278 | hsa-miR-217 | hsa-miR-132 | |
| hsa-miR-625 | hsa-miR-136# | hsa-miR-1254 | hsa-1et-7g# | hsa-miR-888# | hsa-miR-22 | hsa-miR-140-3p | |
| hsa-miR-627 | hsa-miR-141# | hsa-miR-1255B | hsa-miR-222# | hsa-miR-93# | hsa-miR-103 | hsa-miR-15a | |
| hsa-miR-502-3p | hsa-miR-194# | hsa-miR-15b# | hsa-miR-448 | hsa-miR-609 | hsa-miR-365 | hsa-miR-186 | |
| hsa-miR-521 | hsa-miR-206 | hsa-miR-1226# | hsa-miR-376° | hsa-miR-620 | hsa-miR-370 | hsa-miR-887 | |
| hsa-miR-574-3p | hsa-miR-1274A | hsa-miR-124# | hsa-miR-431 | hsa-miR-585 | hsa-miR-328 | hsa-miR-744 | |
| hsa-miR-532 | hsa-miR-340# | hsa-miR-1225-3P | hsa-miR-29b | hsa-miR-1292 | hsa-miR-197 | hsa-miR-125a-3p | 5 |
| hsa-miR-548b | hsa-miR-33a | hsa-miR-1228# | hsa-miR-454 | hsa-miR-548E | hsa-miR-338-3p | hsa-miR-653 | |

6. Non-invasive diagnosis method for the early determination of cancer by means of liquid biopsy according to the preceding claim **wherein** the combination of miRNA with proteins and other markers is the following multi-analytical model combination:
- Multi-analytical model: miR-122, miR-21, miR-223, miR-26a, miR-27a, miR-801, miR-1972, miR-193a-5p, miR-214-3p, miR-365a-3p, miR-92-3p, miR-107, miR-3126-5p, miR-29a, miR-29c, miR-133a, miR-143, miR-145, miR-192, miR-505+AFP, PIVKA-II, AFP-L3, DCP+age+sex; said combinations being selected for detecting a liver tumor state.

7. Non-invasive diagnosis method for the early determination of cancer by means of liquid biopsy according to the preceding claim **wherein** the combination of miRNA with proteins and other markers is the following multi-analytical model combination:
- Multi-analytical model: miR-92a-3p, miR-30b-5p, miR-191-5p, miR-484, miR-328-3p miR-30c-5p miR-374a-5p, let-7d-5p, miR-331-3p, miR-29a-3p, miR-148a-3p, miR-223-3p, miR-140-5p, miR-19b, miR-25, miR-183, miR-21, miR-339-5p, miR-429, miR-205, miR-200b, miR-203, miR-125b, miR-34b, miR-532, miR-628-3p, miR-425-3p, miR-1254, miR-574-5p, miR-182-5p, miR-10a-5p, miR-301b, miR-1244, miR-301a-3p, miR-135b-5p, miR-224-5p, miR-21-5p+CKAP4, CRP, Prolactin, HGF, CEA
said combination being selected for the early detection of a lung tumor.

8. Non-invasive diagnosis method for the early determination of cancer by means of liquid biopsy according to the preceding claim **wherein** the combination of miRNA with proteins and other markers is the following multi-analytical model combination:
- Multi-analytical model: miR-320a-3p, miR-665, miR-3184-5p, miR-6717-5p, miR-4459, miR-6076, miR-3195, miR-1275, miR-3185, miR-4640-5p, miR-200b, miR-200c, miR-191-5p, miR-423-3p, miR-320b, miR-141-3p, miR-625-3p, miR-145-3p, miR-92b-5p, miR-320c, miR-320a-3p, miR-320d+CA125, SPINT1, TACSTD2, CLEC6A, ICOSLG, MSMB, PROK1, CDH3, HE4, KRT19, FR-alpha, CRP, PZ, LCAT+age
said combination being selected for detecting an ovary tumor state.

9. Non-invasive diagnosis method for the early determination of cancer by means of liquid biopsy according to the preceding claim **wherein** the combination of miRNA with proteins and other markers is the following multi-analytical model combination:
- Multi-analytical model: miR-1246, miR-4644, miR-3976, miR-4306, miR-145, miR-150, miR-26b, miR-34a, miR-122, miR-223, miR-505, miR-636, miR-885-5p+CD44v6, Tspan8, EpCAM, MET, CD104, THBS2, CA19-9.
said combination being selected for detecting a pancreas tumor state.

10. Non-invasive diagnosis method for the early determination of cancer by means of liquid biopsy according to the preceding claim **wherein** the combination of miRNA with proteins and other markers is the following multi-analytical model combination:
- Multi-analytical model: miR-16, miR-25, miR-92a, miR-451, miR-486-5p, miR-17-5p, miR-21, miR-106a, miR-106b+CEACAM5, CA9, MSLN, CCL20, SCF, TGF-alpha,MMP-1,MMP-10, IGF-1, CDCP1, PPIA, DDAH-1, HMOX-1, FLI1, IL-7, ZBTB-17, APBB1IP, KAZALD-1, ADAMTS-15,
said combination being selected for detecting a stomach tumor state.
